# EUROPEAN PATENT SPECIFICATION

(11) **EP 2 032 060 B1**
(45) Date of publication and mention of the grant of the patent: **17.04.2013**
(21) Application number: 07798873.1
(22) Date of filing: 21.06.2007
(51) Int. Cl.: A61B 18/18, A61B 18/14

(54) **TORQUE TRANSFER AGENT FOR INTRODUCER**
DREHMOMENTÜBERTRAGUNGSMITTEL FÜR EINE EINFÜHRVORRICHTUNG
AGENT DE TRANSFERT DE COUPLE POUR DISPOSITIF D'INTRODUCTION

(30) Priority: 23.06.2006 US 815853 P; 28.12.2006 US 646528
(43) Date of publication of application: 11.03.2009
(73) Proprietor: St. Jude Medical, Atrial Fibrillation Division, Inc., St. Paul, Minnesota 55117-9913 (US)
(72) Inventor: HOLZBAUR, Michael, East Palo, California 94303 (US); PODMORE, Jonathan L., San Carlos, California 94070 (US)
(74) Representative: Kramer - Barske - Schmidtchen
(86) International application number: PCT/US2007/071760
(87) International publication number: WO 2007/149968

(56) References cited:
- WO-A2-2004/028233
- US-A- 5 607 462
- US-A1- 2002 165 537

## Description

### CROSS-REFERENCE TO RELATED APPLICATIONS

This application claims priority to United States provisional application no. 60/815,853, filed 23 June 2006 (the '853 application). This application also claims priority to United States nonprovisional application no. 11/646,528, filed 28 December 2006 (the '528 application).

### BACKGROUND OF THE INVENTION

### a. Field of the Invention

This invention relates generally to devices and methods for ablating tissue. The diagnosis and treatment of electrophysiological diseases of the heart, and more specifically devices and methods for epicardial mapping and ablation for the treatment of atrial fibrillation, are described in connection with the devices and methods of the present invention. More particularly, the present invention relates to improvements to a sizer and introducer that result in increased torque transfer.

### b. Background Art

It is well known that atrial fibrillation results from disorganized electrical activity in the heart muscle (the myocardium). The surgical maze procedure has been developed for treating atrial fibrillation, and involves the creation of a series of surgical incisions through the atrial myocardium in a preselected pattern so as to create conductive corridors of viable tissue bounded by scar tissue.

As an alternative to the surgical incisions of the maze procedure, transmural ablations of the heart may be used. Such ablations may be performed either from within the chambers of the heart (endocardial ablation), using endovascular devices (e.g., catheters) introduced through arteries or veins, or from outside the heart (epicardial ablation) using devices introduced into the patient's chest. Various ablation techniques may be used, including, but not limited to, cryogenic ablation, radio frequency (RF) ablation, laser ablation, ultrasonic ablation, and microwave ablation. The ablation devices are used to create elongated transmural lesions that is, lesions extending through a sufficient thickness of the myocardium to block electrical conduction forming the boundaries of the conductive corridors in the atrial myocardium. Perhaps most advantageous about the use of transmural ablation rather than surgical incision is the ability to perform ablation procedures without first establishing cardiopulmonary bypass (CPB).

In performing the maze procedure and its variants, whether using ablation or surgical incisions, it is generally considered most efficacious to include a transmural incision or lesion isolating the pulmonary veins from the surrounding myocardium. The pulmonary veins connect the lungs to the left atrium of the heart, joining the left atrial wall on the posterior side of the heart. Such procedures have been found to offer 57% to 70% success without antiarrhythmic drugs. However, they are also associated with a 20% to 60% recurrence rate as the result of lesion recovery, non-pulmonary vein foci of the arrhythmia, or the need for further tissue modifications.

Currently, techniques to isolate the pulmonary veins can involve the use of devices designed to curve around the circumference of the pulmonary veins and left atrial tissue. Accordingly, many catheters, introducers, or other devices used have a defined curved region. Placing these devices through a lateral thoracotomy incision can be challenging and time consuming because of the small space in which the surgeon must operate. Thus, there is a need for devices to safely and more efficiently direct these curved devices around an anatomical feature, such as the atrium or pulmonary veins, during the surgical procedure.

WO 2004/028233 A1 discloses the most relevant prior art.

The invention is defined in claim 1.

It is therefore desirable to be able to provide an introducer for introducing an ablation device that is more effective in placing the ablation device in the area to be treated.

It is also desirable to provide an introducer that is more capable of manipulation by a surgeon to navigate a patient's vasculature.

Disclosed herein is a kit for use in a lateral thoracotomy procedure. The kit includes an introducer that has an elongated flexible body having a proximal end and a distal end, and an intermediate portion therebetween, wherein the distal end forms at least a partial loop when in a resting, unbiased (i.e., relaxed) state. The elongated flexible body also includes a lumen extending from the proximal end to a point between the proximal end and a point at which the distal end begins to curve, as well as at least one torque imparting receptacle that is accessible via the lumen. The kit also includes a torque transfer device that has a handle and a shaft fixedly coupled to the handle. The shaft is configured to be inserted into the lumen and further into the at least one receptacle. When the user imparts torque to the handle, the shaft transfers the torque to the intermediate portion of the introducer through the receptacle. The introducer may also have a connector that is configured to receive the handle of the torque transfer device. Optionally, the proximal end of the introducer may include a snap-fit connector that is configured to receive the handle of the torque transfer device. Preferably, the shaft of the torque transfer device is made of a material that resists twisting forces, such as steel. The elongated flexible body of the introducer may have two or more receptacles, such that a shaft may be configured with a corresponding number of distal ends to fit within the receptacles. The cross section of the elongate body may vary, and is preferably larger at the proximal end than the distal end. The elongate body of the introducer may also include braided wire assembly to help transfer torque from the proximal end to the distal end.

Also disclosed is a sizing device for sizing at least a portion of a circumference around at least one pulmonary vein along an epicardial surface. The sizing device includes an elongated body having a distal end, a proximal end, and an intermediate portion therebetween, and the elongated body includes at least a partial loop near the distal end when in a relaxed state. The sizing device also includes a handle at the proximal end, and the elongated body includes a lumen extending from the proximal end to a point within the intermediate portion. The elongated flexible body also includes at least one receptacle accessible via the lumen, and the receptacle is configured to impart torque onto the intermediate portion of the elongated body. The sizing device also includes a shaft fixedly coupled to the handle and extending from the handle to the at least one receptacle to assist in transferring torque from the handle to the intermediate portion. The sizing device preferably includes a plurality of sizing indicators along a length of the elongated body, wherein the plurality of sizing indicators are indicative of a distance that is being measured by the at least a partial loop that is formed near the distal end of the sizing device when the sizing device is placed about a portion of a heart.

Also disclosed is a sizing device for sizing an area around one or more pulmonary veins along an epicardial surface. The sizing device includes an elongated body having a distal end, a proximal end, and an intermediate portion therebetween. The distal end is configured to form at least a partial loop when in a resting, unbiased shape, and the proximal end has a larger cross section than the distal end. The elongated body has an insert located at either the distal end or the intermediate portion. A handle is located at the proximal end of the elongated body, and a shaft is rigidly coupled to the handle and extends to the insert of the elongated body to assist in transferring torque from the handle to at least one of the distal end and the intermediate portion of the elongated body. The handle may be a snap-fit connector that is configured to receive an ablation device. The shaft is preferably made of a material that resists twisting forces, such as steel. The insert may be formed as a groove, such as on the head of a screw, and the shaft may have a flattened head, similar to a screwdriver, which mates with the grooved insert.

Also disclosed is a method of introducing an ablation device for ablating an epicardial surface. The method includes the steps of: providing an introducer having an elongated body with a distal end, a proximal end, and an intermediate portion therebetween, the distal end forming at least a partial loop in a relaxed state; inserting a torque transfer device into the introducer, the torque transfer device including a handle located at a proximal end and a shaft that extends from the handle; inserting the introducer through an incision in a patient; manipulating the introducer via the proximal end to wrap the distal end of the introducer around at least a portion of a heart; removing the torque transfer device; attaching an ablation device to the proximal end of the introducer; and manipulating the introducer to position the ablation device about at least a portion of the patient's heart. The introducer may also be used estimate a measurement that is indicative of a circumference about at least a portion of the patient's heart. For example, the measurement may tell the user how many ablation elements are needed to encircle the portion of the patient's heart, such as to form a pulmonary vein isolation ablation lesion. Based on the measurement, the user may select an appropriate sized ablation device.

The device of the present invention enables the selection and introduction of ablation devices to create a uniform, continuous, linear lesion during cardiac ablation. The sizer/introducer device can be better manipulated in part because of the increased ability to effect torque transfer from the proximal handle to the distal end of the introducer.

An advantage of the present invention is that less time is needed to size and to introduce the ablation device in the tissue area to be treated.

Another advantage of the present invention is that smaller incisions may be used by a surgeon during ablation treatment, which speeds the recovery process for the patient.

The aspects, features, details, utilities, and advantages of the present invention will be apparent from reading the following description and claims, and from reviewing the accompanying drawings.

### BRIEF DESCRIPTION OF THE DRAWINGS

Fig. 1 schematically illustrates an ablation system.

Fig. 2 shows an introducer.

Fig. 3 is a side view of the introducer illustrated in Fig. 2.

Fig. 4 illustrates an ablation device for creating PV isolation ablations.

Fig. 5 illustrates the ablation device of Fig. 4 in an open position.

Fig. 6 shows the ablation device of Fig. 4 forming a closed loop.

Fig. 7 illustrates the introducer of Fig. 2 being advanced around the pulmonary veins.

Fig. 8 depicts the introducer extending around the pulmonary veins in order to size an ablation device.

Fig. 9 shows the ablation device being connected to the introducer.

Fig. 10 illustrates the ablation device coupled to the introducer and being advanced around the pulmonary veins via manipulation of the introducer.

Fig. 11 illustrates the same thing as Fig. 10 at a later stage of the process.

Fig. 12 shows the introducer being decoupled from the ablation device.

Fig. 13 is an expanded view of the connection between the introducer and the ablation device.

Fig. 14 depicts the ablation device forming a closed loop about the pulmonary veins.

Fig. 15 depicts the ablation device forming a closed loop about the pulmonary veins and secured in this configuration using sutures.

Fig. 16 is an illustration of various components including the introducer/sizer and torque transfer device.

Fig. 17 is a cross sectional end view of the introducer taken near the proximal end.

### DETAILED DESCRIPTION OF THE INVENTION

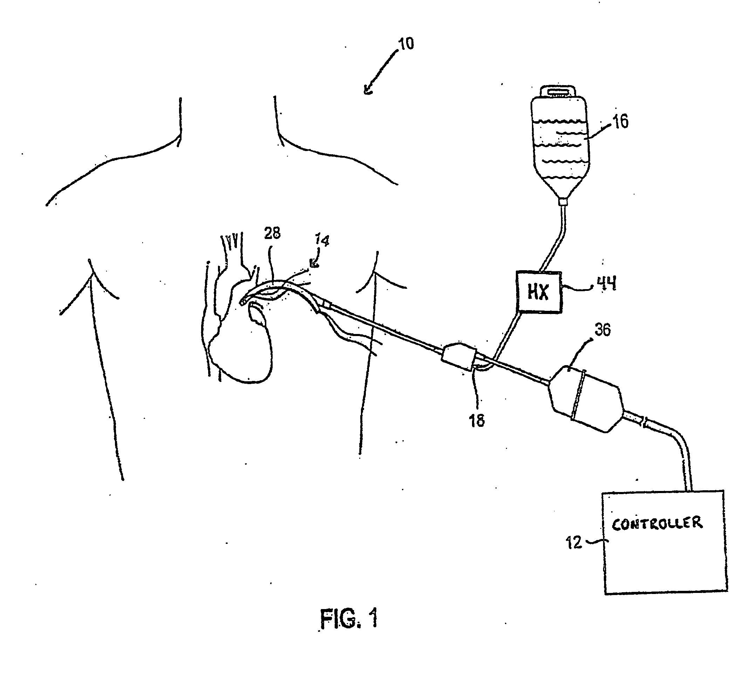

Referring now to Fig. 1, an ablation system 10 is shown. Ablation system 10 includes a controller 12, which preferably operates to deliver focused ultrasound energy. Ablation system 10 may be used to wrap an ablation device 14 around the pulmonary veins at an epicardial location in order to create a pulmonary vein (PV) isolation ablation lesion. Ablation system 10 may further include a source 16 of a flowable material, which may be a bag of saline that provides a gravity feed to ablation device 14 via a standard luer connection 18.

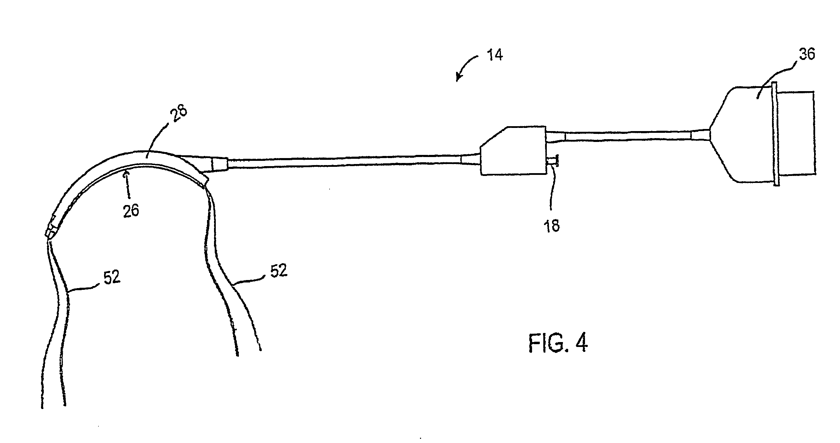

With reference to Figs. 4-6, ablation device 14 includes a plurality of ablation elements 26 substantially aligned along a common axis and coupled together, preferably through integrally formed hinges in ablation device 14. By "substantially aligned along a common axis," it is meant that there is little or no staggering between ablation elements 26 along the direction in which they are coupled together. It should be understood that ablation elements 26 may alternatively be coupled together with mechanical connections, rather than integrally formed hinges.

Ablation device 14 preferably has from about 5 to about 30 ablation elements 26, more preferably from about 10 to about 25 ablation elements 26, and most preferably less than about 15 ablation elements 26. It should be understood, however, that any number of ablation elements 26 may be used depending upon the specific application for ablation device 14. For example, ablation device 14 may be used to extend around only a single vessel, such as the aorta, a pulmonary vein, the superior vena cava, or inferior vena cava, in which case ablation device 14 preferably includes about 4 to about 12 ablation elements 26, and more preferably includes about 8 ablation elements 26. Each ablation element 26 is preferably a discrete, autonomously controlled cell.

A body 28 of ablation device 14 is preferably made of a polymeric material such as polycarbonate, polyetherimide (e.g., Ultem®), silicone, or urethane, and is preferably formed by injection molding. One of ordinary skill will appreciate, however, that any suitable materials and methods may be used to form ablation device 14. Preferably, an outer surface of body 28 is smooth in order to limit the risk of catching ablation device 14 on patient tissue or otherwise causing trauma during insertion of ablation device 14.

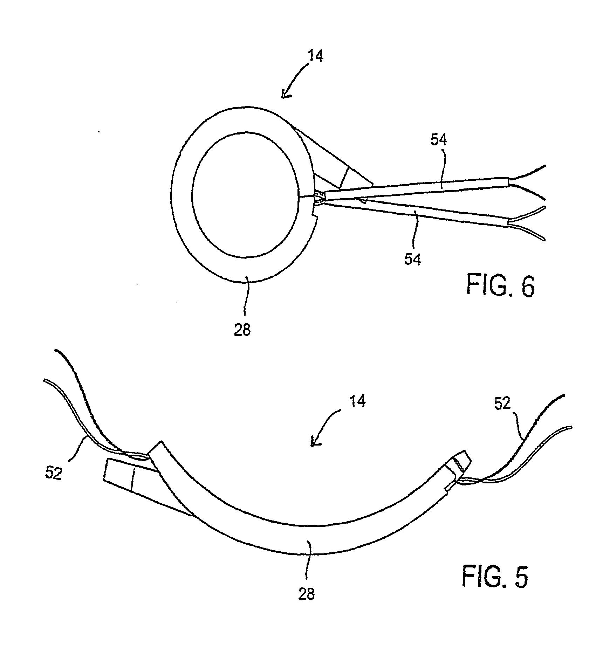

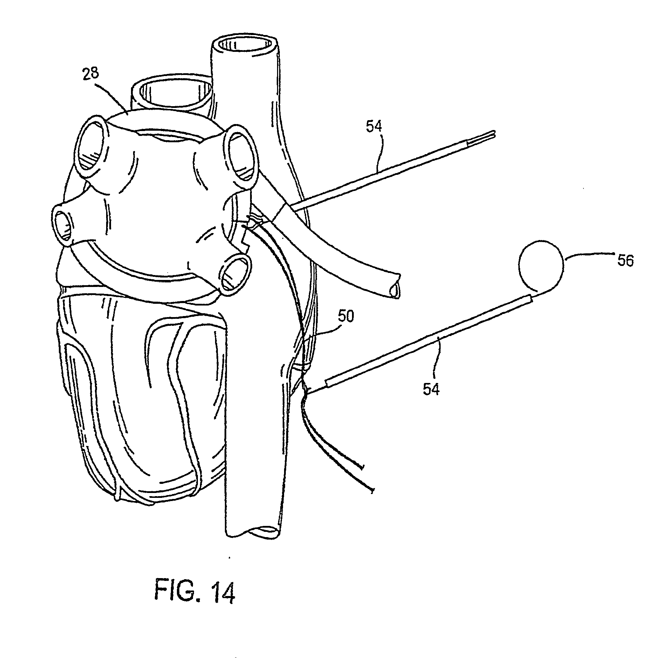

Ablation device 14 is configured to have a predetermined curvature that facilitates encircling an area of the heart while simultaneously permitting ablation device 14 to be straightened or flattened to minimize the overall width thereof. The latter (i.e., flattened) configuration facilitates insertion of ablation device 14 through a relatively smaller incision in the patient in order to reach the heart tissue, and thus is referred to herein as an "insertion configuration." In other words, ablation device 14 is configured to permit at least two distinct configurations: a predetermined curvature (e.g., Fig. 5) to facilitate manipulation around the heart and a substantially straight, generally flattened shape (having little or no curvature) to facilitate insertion into the patient's body. By using the flattened configuration during insertion, the surgeon may use a smaller incision, which reduces the patient's recovery time. By using the curved configuration to manipulate ablation device 14 around the patient's heart, the surgeon is able to more easily maneuver ablation device 14 into position for treatment. Ablation device 14 may also be deformed into a third configuration, which is a generally closed loop as seen in Figs. 6, 14, and 15. This third configuration will be described in further detail below.

The phrase "predetermined curvature" is intended to convey that ablation device 14 is designed to assume a curved shape and maintain that general shape during certain intended manipulations. For example, while ablation device 14 may be maintained in a substantially straightened position for insertion, ablation device 14 is intended to resume and maintain a curved shape during manipulation about the heart. Additional forces may be applied on ablation device 14 in order to increase or decrease the degree of curvature, for example into the substantially closed loop third configuration illustrated in Fig. 6. The use of "predetermined" is intended to convey that ablation device 14 maintains a generally curved shape while being positioned around a portion of the heart (that is, the "relaxed" state of ablation device 14, with no external forces applied thereto, is a generally curved configuration).

In one preferred embodiment of ablation device 14, ablation elements 26 are connected using a superelastic material, including, by way of example only, a memory metal such as Nitinol. As one of ordinary skill in the art will understand, a "superelastic material" is a material that can be subjected to very large strains without plastic deformation. The superelastic properties allow ablation device 14 to be substantially deformed to become substantially coplanar and then to return to the predetermined curvature. For example, all ablation elements 26 may be interconnected using one or more strands of Nitinol, or another superelastic material, such that ablation device 14 may be substantially straightened for insertion into the patient through a relatively small incision, and thereafter manipulated into position about the heart in a generally curved configuration. The Nitinol or other superelastic material may take the form of a hinge wire that connects a plurality of ablation elements 26 to maintain the predetermined curvature.

In one embodiment, each ablation element 26 is contained in a housing, the edges of which may be angled to permit adjacent ablation elements 26 to have at least two relationships to one another: one in which they are substantially coplanar, resulting in a substantially flat configuration, and another in which they are at an angle, resulting in a generally curved configuration. Preferably, the angle between the faces of adjacent ablation elements 26 when ablation device 14 is in its relaxed state (i.e., the generally curved configuration) may be adjusted based on the number of ablation elements 26, and may typically be between about 10 degrees and about 30 degrees. The hinges may be integrated wholly or partially into the housings.

It is also contemplated that the adjustable configurations of ablation elements 26 may be implemented utilizing a spring system, such as a combination of mechanical hinges and/or springs. The mechanical hinges and/or springs may be used in conjunction with ablation elements 26 having angled edges 30 as described above. In addition, a standard guidewire structure (not shown), which generally includes a tightly coiled wire and, optionally, a core wire running therethrough, may be utilized to interconnect ablation elements 26.

Optionally, ablation device 14 may be deformed temporarily during insertion of ablation device 14 into the patient with the assistance of a sheath. The sheath applies a deforming force to ablation device 14 and assists in maintaining ablation elements 26 in a substantially straight insertion configuration. Preferably, the sheath is a straight cylinder that is sized to accommodate ablation device 14 in the substantially straight insertion configuration. Thus, the sheath may be used to introduce ablation device 14 through an incision into the patient. Once ablation device 14 has been introduced through the incision, the sheath may be removed, and the tension caused by the superelastic wire or spring system will cause ablation device 14 to resume its predetermined curvature.

Alternatively, a stylet may be used to deform ablation device 14 into the generally straight insertion configuration. Each ablation element 26 may include a guide tube shaped to receive the stylet therethrough. The guide tube may be internal to each ablation element 26 or mounted to the exterior of ablation device 14. As the stylet passes through the guide tubes, it applies a deforming force to ablation device 14 and assists in maintaining ablation elements 26 in a substantially straight configuration to facilitate insertion of ablation device 14 through an incision into the patient. Once ablation device 14 has been introduced, the stylet may be withdrawn, at which time the restorative force caused by the superelastic wire or spring system will cause ablation device 14 to resume its predetermined curvature.

Ablation elements 26 may be any element for directing and delivering ablating energy to the cardiac tissue, including, but not limited to, focused ultrasound elements, radio frequency (RF) elements, laser elements, and microwave elements. Ablation elements 26 preferably have a width of about 1 mm to about 15 mm, and more preferably of about 10 mm, and a length of about 2 mm to about 25 mm, and more preferably of about 12 mm. When an RF element is used, the RF element is coupled to an RF generator which transmits RF energy to the element. The RF element is preferably a stainless steel or gold plated copper electrode, although any suitable electrode may be used. For RF ablation elements 26, ablation elements 26 are preferably spaced apart from the target tissue, by a distance of between about 0.5 mm to about 3 mm, and more preferably by about 1.5 mm.

Ablation elements 26 are coupled to controller 12 via wires. The wires may be collectively incorporated into a plug 36 usable to couple ablation device 14 to controller 12 as shown in Fig. 1. Controller 12 controls ablation, for example in the manner described herein. A source of ablation energy (e.g., a signal generator) may be part of controller 12 or separate therefrom. One or more temperature sensors, preferably thermocouples or thermistors, are positioned within recesses in the inner and outer lips of ablation device 14 in order to measure temperature. The temperature sensors are also coupled to controller 12, for example via plug 36, for monitoring purposes and to provide temperature feedback for controlling the ablation process as described herein.

Each ablation element 26 may also have a membrane that contains the flowable material within a fluid chamber to provide a conformable interface with the tissue to be ablated. The membrane may include openings through which the flowable material may leak or weep, and each membrane may be fed by an individual inlet leading thereto.

The flowable material is preferably supplied at an average flow rate of at least about 0.24 cc/sec, more preferably at least about 0.50 cc/sec, and most preferably at least about 1.0 cc/sec to each ablation element 26, although lower or higher flow rates may be used. The flowable material is preferably delivered to the inlet of ablation device 14 at a set pressure that results in the desired average flow rate through ablation elements 26. The flowable material may be heated or cooled as desired or required by passing it through a heat exchanger 44 prior to delivery to the inlet of ablation device 14 (e.g., luer connection 18 as seen in Fig. 1). The flowable material is preferably delivered at a temperature of no more than about 40 degrees C, and more preferably at a temperature of no more than about 25 degrees C, to cool the tissue and/or ablation elements 26. A fluid permeable, porous structure, such as gauze, may be also positioned to hold the flowable material within the fluid chamber and prevent direct contact between ablation elements 26 and the tissue being ablated.

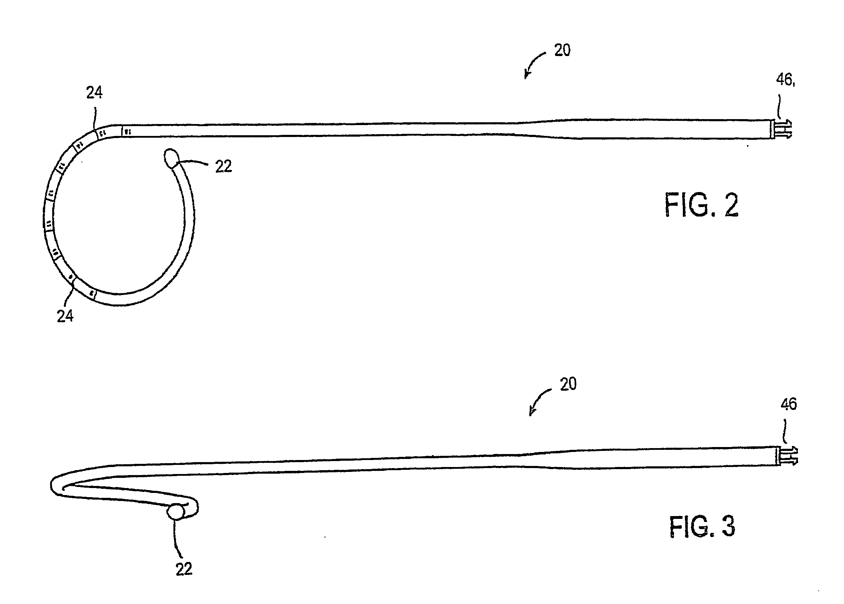

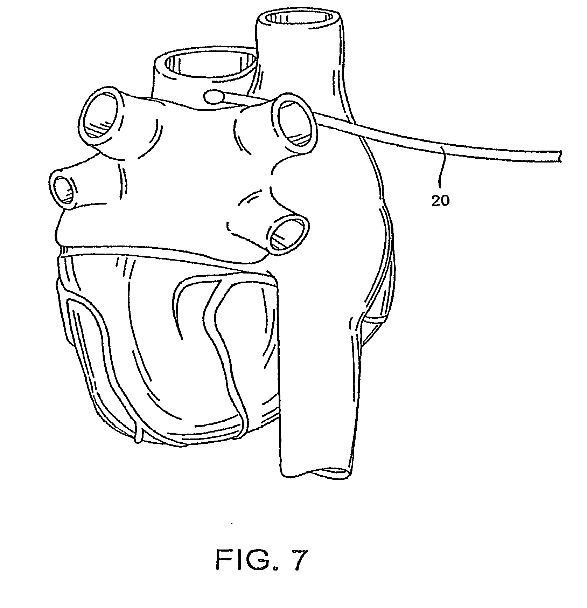

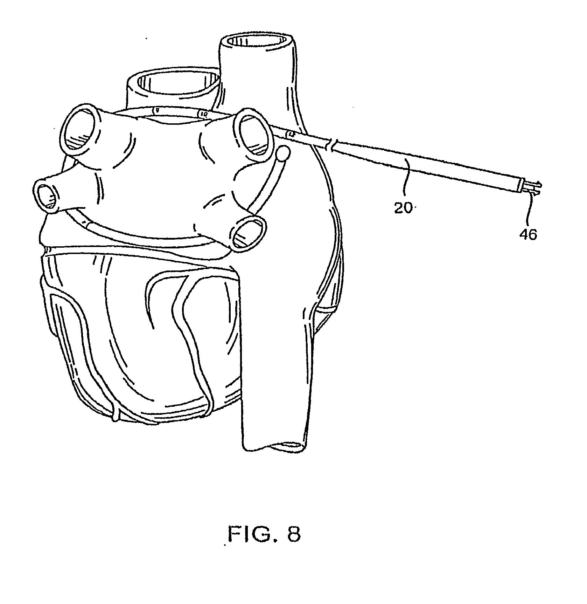

The system further includes an introducer 20, illustrated in Figs. 2 and 3, which is advanced around the pulmonary veins as shown in Figs. 7 and 8 and described below. As shown in Fig. 2, introducer 20 preferably forms a substantially closed loop in an unbiased configuration, with a small offset near its distal tip 22 as shown in Fig. 3.

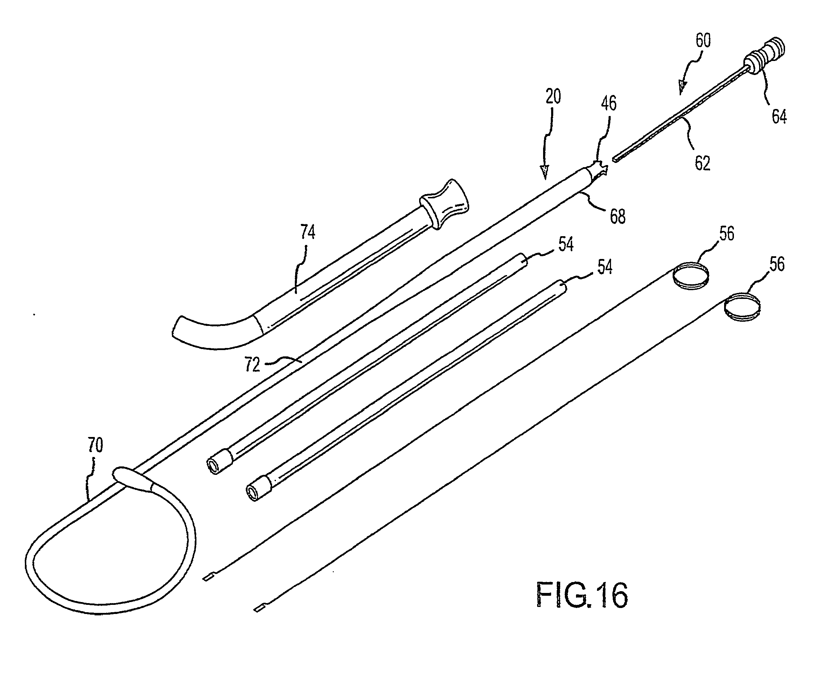

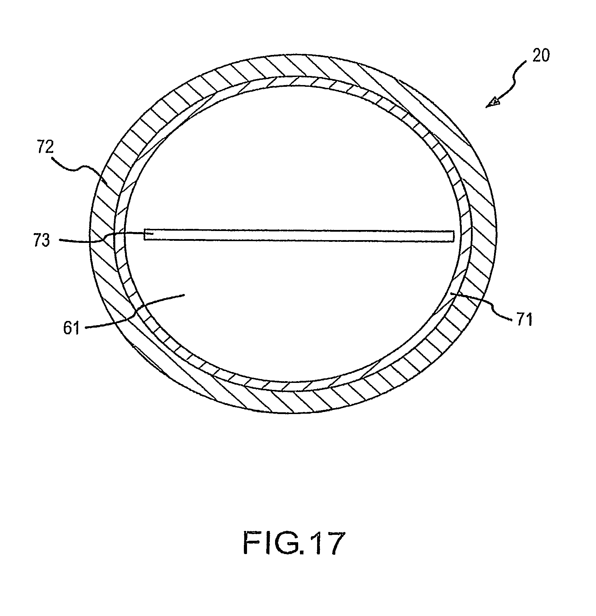

Fig. 16 depicts introducer 20 and torque transfer device 60, which includes a rigid shaft 62 that is designed to be inserted into a lumen 61 (Fig. 17) of introducer 20. Preferably, torque transfer device 60 also includes a handle 64 on the proximal end. More preferably, handle 64 interlocks with a corresponding connection 46 on the proximal end of introducer 20. Preferably, shaft 62 of torque transfer device 60 is made of a material that resists twisting forces, such as steel or stainless steel. Of course, any relatively rigid material may be used, including, without limitation, other metals and composite materials.

Introducer 20 has an elongated flexible body having a proximal end 68, a distal end 70, and an intermediate portion 72 therebetween. Distal end 70 is configured to form at least a partial loop when in a resting, unbiased shape. That is, the relaxed configuration of distal end 70 is at least a partial loop. The elongated flexible body also includes lumen 61 extending from proximal end 68 to a point between proximal end 68 and the point at which distal end 70 begins to curve. Lumen 61 is designed to accommodate shaft 62 of torque transfer device 60.

The elongated flexible body of introducer 20 preferably contains at least one receptacle 73 (Fig. 17) or insert, accessible via lumen 61, to accommodate the distal end of shaft 62 of torque transfer device 60. Receptacle 73 may take the form of a groove on the head of a screw, and shaft 62 may have a flattened head like a screwdriver, such that shaft 60 fits into receptacle 73 like a screwdriver fits into the head of a screw. Of course, receptacle 73 may take other forms, but generally is designed to mate with the distal end of shaft 60. The elongated flexible body may also have a plurality of such receptacles.

The location of receptacle 73 may vary at any point between proximal end 68 of introducer 20 and the point at which distal end 70 begins to curve. Preferably, receptacle 73 is located at least one-third of, and more preferably at least half of, the distance between proximal end 68 and the point at which distal end 70 begins to curve. Where the cross section of introducer 20 varies, as depicted in Fig. 16, it is preferred that receptacle 73 be located such that shaft 62 of torque transfer device 60 penetrates into lumen 61 of introducer 20 at least as far as the point at which the cross section begins to decrease.

Optionally, proximal end 68 of introducer 20 may include a connector, such as snap-fit connector 46, that is configured to receive handle 64 of torque transfer device 60. This helps to maximize torque transfer from handle 64 to proximal end 68 of introducer 20.

The use of torque transfer device 60 permits the surgeon to have greater control during the process at which introducer 20 is inserted into the patient's body, and thus improves the ability of the surgeon to quickly advance introducer 20 and size the patient for an ablation device 14. Torque transfer agent 60 may then be removed to restore flexibility to introducer 20, thereby facilitating the introduction and placement of ablation device 14. The improved stiffness (due to the insertion of torque transfer device 60) gives the surgeon improved control in the beginning of the procedure, and the increased flexibility (due to the removal of torque transfer device 60) gives the surgeon improved control in the treatment phase of the procedure.

Introducer 20 may be made using polymers, including, without limitation, etched polytetrafluoroethylene (PTFE), polyether block amides, nylon and other thermoplastic elastomers. Once such elastomer is Pebax® made by Arkema, Inc. Pebax of various durometers may also be used, including, without limitation, Pebax 30D to Pebax 70D. One of ordinary skill in the art will select the components to use in making introducer 20 based on the desired level of flexibility that is needed for a specific application of ablation system 10.

A braided wire assembly 71 (Fig. 17) may be used in at least a portion of introducer 20. Braided wire assembly 71 preferably extends from proximal end 68 towards distal end 70. For example, it may extend from proximal end 68 to the depth at which torque transfer device 60 is inserted into introducer 20. Braided wire assembly 71 may be formed of stainless steel wire, including for example 0.003" high tensile stainless steel wire.

The braided wire assembly may be formed in a standard braid pattern, for example, 16 wires at 45-60 picks per inch ("PPI"). Alternatively, the braided wire assembly may have a varying braid density. For example, the braided wire assembly may be characterized by a first braid density at proximal end 68 of introducer 20 and then transition to one or more different braid densities approaching distal end 70 of introducer 20. The braid density at distal end 70 may be greater or less than the braid density at proximal end 68. In a specific example, the number of picks per inch at the base (proximal end) is about 50 and the number of picks per inch at the depth at which the torque transfer device is inserted is about 10. Preferably, the braided wire assembly is used in conjunction with torque transfer device 60, though it is also contemplated that the braided wire assembly may be used instead of torque transfer device 60.

Introducer 20 may be used as a sizing device for sizing ablation device 14. For example, as shown in Fig. 2, introducer 20 may have size indicators 24 usable to determine the appropriate size of ablation device 14. For ablation device 14 shown in Figs. 4 through 6 and described in detail herein, the size of ablation device 14 is effectively determined by the number of ablation elements. It is also contemplated, however, that other methodologies for sizing ablation device 14 may be used.

In use, and as illustrated in Figs. 7 and 8, introducer 20 is inserted into the patient and passed through an incision in the pericardial reflection adjacent the right superior pulmonary vein adjacent the transverse pericardial sinus. Preferably, introducer 20 is inserted in a generally straight configuration. Thus, a straightening introducer 74 (Fig. 16) may be employed to good advantage in the insertion of introducer 20 into the patient.

Once inserted into the patient, introducer 20 may be advanced through the transverse pericardial sinus, around the left superior and inferior pulmonary veins, and out through another incision in the pericardial reflection near the right inferior pulmonary vein. The appropriate size of ablation device 14 may then be read using indicators 24 imprinted on introducer 20. For example, in Fig. 8, size indicators 24 of introducer 20 read "12," indicating that an ablation device 14 having 12 ablation elements will substantially encircle the pulmonary veins.

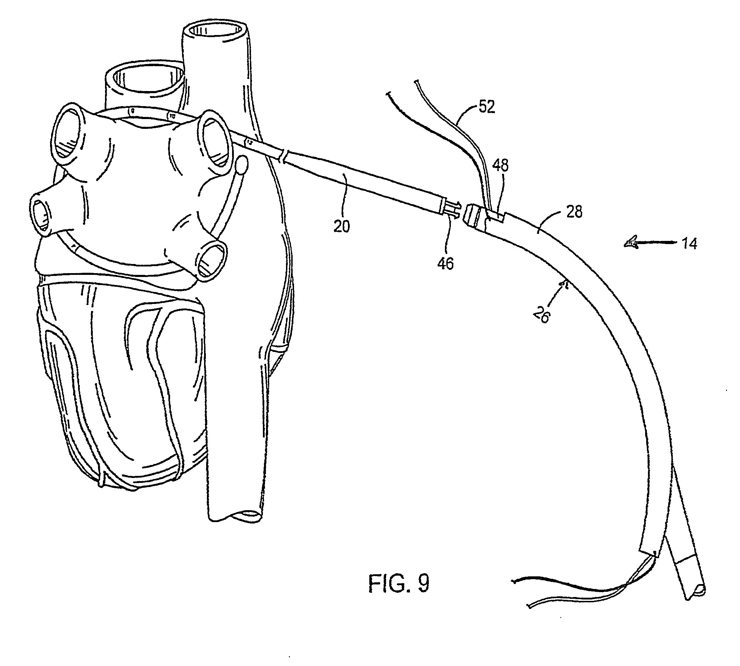

After the appropriate size of ablation device 14 is identified, for example by using introducer 20 as described above, ablation device 14 may be coupled to proximal end 68 of introducer 20 with any suitable connection, such as mating snap fit connectors 46 described above and shown in Figs. 9, 13, and 16. It should be understood that the appropriate size of ablation device 14 may also be determined using a device or method independent of introducer 20. Ablation device 14 is preferably introduced into the patient while straightened, optionally through the use of a sheath, stylet, or other straightening device.

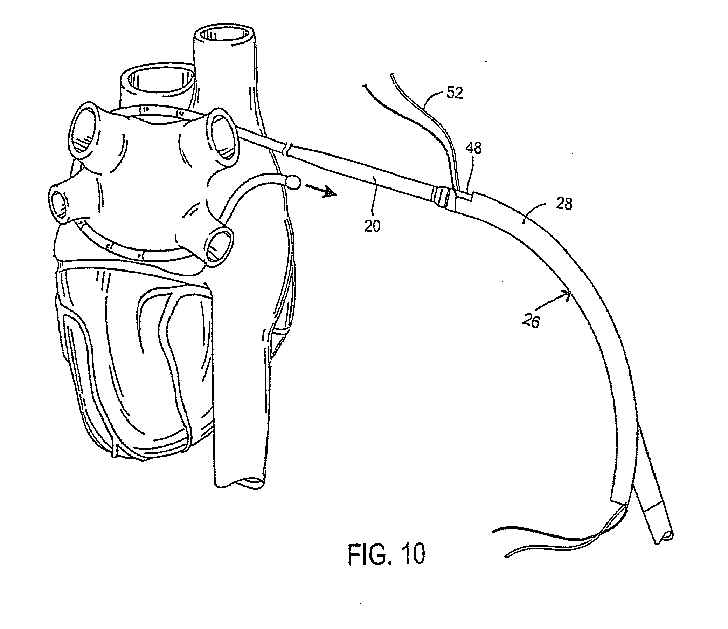

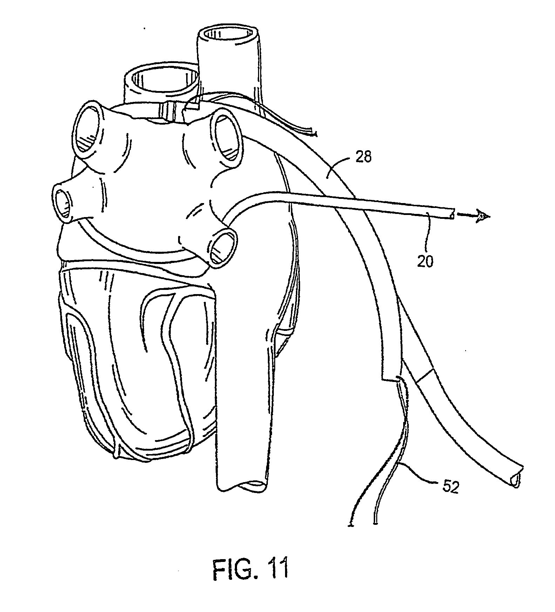

Introducer 20 is then pulled further, as shown in Figs. 10 and 11, in order to manipulate ablation device 14 and wrap ablation device 14 about the pulmonary veins. Once ablation device 14 has been introduced through the incision, the sheath, stylet, or other straightening device may be removed in order to permit ablation device 14 to resume its predetermined curvature for manipulation about the pulmonary veins.

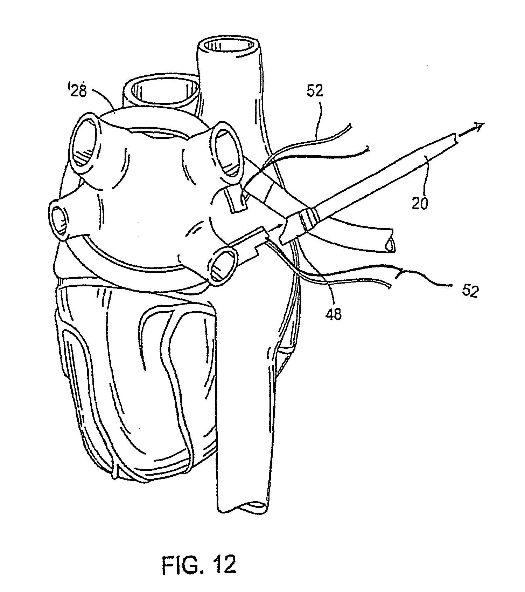

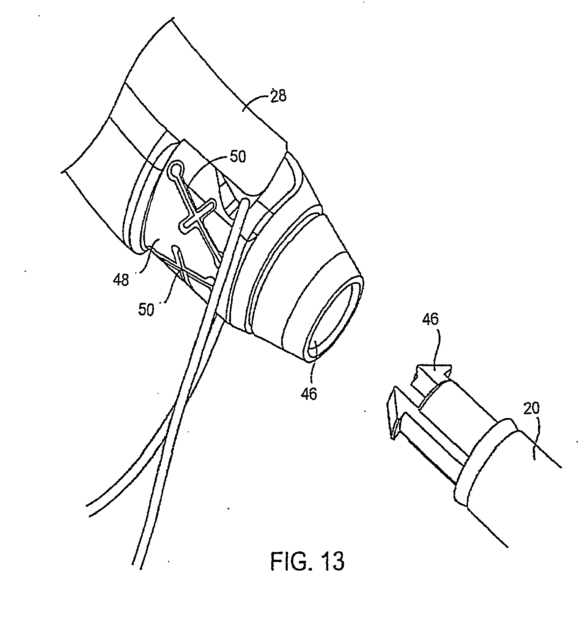

As shown in Fig. 12, once ablation device 14 is wrapped about the pulmonary veins, introducer 20 may be detached from ablation device 14 by detaching a releasable assembly 48 from ablation device 14. In some embodiments, releasable assembly 48 is detached by simply cutting one or more sutures 50 (Fig. 13) that hold releasable assembly 48 to the device 14. It is also contemplated that snap fit connection 46 between introducer 20 and ablation device 14 may be releasable to permit decoupling introducer 20 at the same place introducer 20 is initially coupled to ablation device 24 without the need to cut one or more sutures 50.

Ablation device 14 may then be locked to itself in a substantially closed-loop configuration to encircle all or part of the pulmonary veins. Device 14 has elongate elements, such as sutures 52, at both ends, which can be tensioned and cinched together to lock the ends of device 14 to each other using tourniquets 54 and suture snares 56 as shown in Figs. 6, 14, 15, and 16.

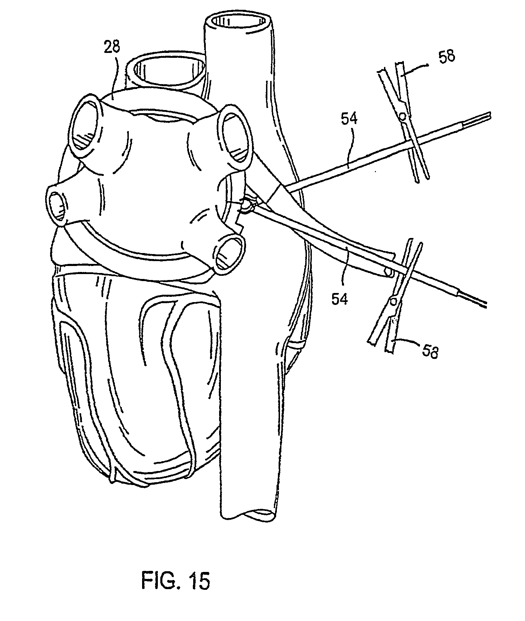

Preferably, ablation device 14 has two opposing pairs of sutures 52, though other numbers and configurations of sutures 52 are possible. Sutures 52 are tensioned using tourniquets 54 to approximate the ends of ablation device 14, such that tensioning sutures 52 forces the ends of ablation device 14 together. The sizing of ablation device 14 (which may be determined using introducer 20, as described above) provides a snug fit around all or part of the pulmonary veins such that tensioning sutures 52 forces ablation device 14 into contact with the epicardial surface. Hemostats 58 or other suitable devices may be used to pinch or crimp tourniquets 54 in order to secure ablation device 14 in place about the pulmonary veins as seen in Fig. 15. Alternatively, ablation device 14 may utilize a locking mechanism, such as a buckle or other releasable locking mechanism, to be locked to itself and thereby secured in place about the pulmonary veins.

Ablation device 14 may also contain a suction well to assist device 14 in adhering to the tissue to be ablated. The suction well may take any form, and is preferably formed between the inner and outer lips of body 28 of ablation device 14. The suction well may have a suction port coupled to a vacuum source through a lumen. The vacuum source may be activated to cause the suction well to hold ablation element 26 against the tissue to be ablated. The suction port preferably has a cross-sectional size that is no more than about 10% of the cross-sectional size of the lumen. Thus, if suction is lost at one ablation element 26, suction can be maintained at other ablation elements 26, since the relatively small suction port produces low flow. Of course, another part of the vacuum flow path, other than the suction port, may be sized small to reduce losses through ablation elements 26 not adhered to the tissue.

Controller 12 preferably activates ablation elements 26 in a predetermined manner. The phrase "predetermined manner" is intended to refer to a non-random sequence. In one mode of operation, ablation is carried out at adjacent ablation elements 26. Ablation may also be carried out at a number of pairs of adjacent ablation elements 26, such as the first and second ablation elements 26 and the fifth and sixth ablation elements 26. After ablation is carried out at these adjacent ablation elements 26, another pair or pairs of adjacent ablation elements 26 are activated, such as the third and fourth and seventh and eighth ablation elements 26. The continuity of the ablation between adjacent ablation elements 26 may be confirmed in any suitable manner. In other modes of operation, controller 12 may energize every other ablation element 26, every third ablation element 26, or a limited number of ablation elements 26, such as no more than four. Controller 12 may also activate less than about 50%, and even less than about 30%, of the total ablation area at one time (for ablation device 14, a percentage of the total ablation area is effectively a percentage of the total number of ablation elements 26).

Preferably, ablation device 14 is designed to achieve and maintain particular near surface (NS) temperatures during an ablation procedure. For example, ablation device 14 may be designed to maintain a near surface (NS) temperature of about 0 degree C to about 80 degrees C, more preferably about 20 degrees C to about 80 degrees C, and most preferably about 40 degrees C to about 80 degrees C. The temperature can be adjusted by changing the flow rate of the flowable material, the temperature of the flowable material, and/or the power delivered to ablation elements 26.

In some embodiments, ablation is controlled based on temperature measured by the temperature sensors. For example, controller 12 may incorporate a multiplexer that delivers ablating energy only to those ablation elements 26 having a temperature below a threshold temperature. Alternatively, the multiplexer may deliver ablating energy only to the coldest ablation elements 26 or only to those ablation elements registering the coolest temperatures.

After measuring the temperature change over time, the temperature response may be analyzed to determine the appropriate ablation technique. The analysis may be a comparison of the temperature response to temperature response curves of known tissue types. The temperature response curves may be developed empirically or may be calculated. The temperature response may also consider other variables input by the user, including, but not limited to, blood temperature, blood flow rate, and the presence and amount of fat. When assessing the temperature response during heating with ablation elements 26, the amount of energy delivered to the tissue may also be taken into account in characterizing the tissue.

Using the results of the temperature response assessment, controller 12 preferably determines the appropriate ablation technique to produce the desired far surface (FS) temperature. In one mode of operation, controller 12 determines the amount of time required to reach a desired FS temperature when the NS is maintained at a temperature of less than about 60 degrees C. Controller 12 preferably maintains an adequate flow rate and temperature of the flowable material to maintain the desired NS temperature. Controller 12 monitors the temperature of the NS with the temperature sensors. After the calculated amount of time has elapsed, controller 12 automatically stops delivering ablating energy to ablation elements 26. Alternatively, the ablation may take place until the NS reaches a target temperature as sensed by the temperature sensors. The continuity of the ablation may then be checked in any manner described herein.

Ablation device 14 preferably delivers ultrasound energy focused in at least one dimension. In particular, ablation device 14 preferably delivers focused ultrasound having a focal length of about 2 mm to about 20 mm, more preferably of about 2 mm to about 12 mm, and most preferably of about 8 mm. Stated another way, a focus is spaced apart from a bottom (or contact) surface of ablation device 14 along a focal axis (FA) within the stated ranges. The focused ultrasound also forms an angle of about 10 degrees to about 170 degrees, more preferably of about 30 degrees to about 90 degrees, and most preferably of about 60 degrees relative to the FA. Preferably, a piezoelectric transducer is utilized as an ultrasonic ablation element 26. The transducer is preferably mounted within a housing having an enclosure and a top that fits over the enclosure. The enclosure may have curved lips on both sides of the enclosure that generally conform to the curvature of the transducer. The transducer preferably has a length of about 0.43 inch, a width of about 0.35 inch, and a thickness of about 0.017 inch. The transducer has a radius of curvature (R) consistent with the preferred focal lengths described above. The transducer forms an angle (A) with the focus (F) within the preferred angle ranges described above.

An advantage of using focused ultrasonic energy is that the energy can be concentrated within the tissue. Another advantage of using focused ultrasound is that the energy diverges after reaching the focus, thereby reducing the possibility of damaging tissue beyond the target tissue as compared to collimated ultrasonic energy. When ablating epicardial tissue with collimated ultrasound, the collimated ultrasound energy not absorbed by the target tissue travels through the heart chamber and remains concentrated on a relatively small area when it reaches the endocardial surface on the other side of the chamber. The present invention reduces the likelihood of damage to other structures since the ultrasonic energy diverges beyond the focus and is spread over a larger area.

Although the focused ultrasonic energy is preferably produced with a curved transducer, the focused ultrasonic energy may be produced with any suitable structure. For example, acoustic lensing may be used to provide focused ultrasound. The acoustic lens can be used with a flat piezoelectric element and matching layer. Furthermore, although the ultrasound energy is preferably emitted directly toward the tissue, the ultrasound energy may also be reflected off a surface and directed toward the tissue.

The energy may also be produced by a number of small transducers oriented to focus or concentrate ultrasonic energy, such as at least about 90% of the energy, within the preferred angle ranges and radius of curvature described herein when viewed along a longitudinal axis or along the FA. For example, a multi-element acoustic phased array may be used to provide an acoustic beam-steering capability from one or more cells. One skilled in the art can also appreciate the use of multiple matching layers, focusing acoustic lenses, and non-focusing acoustic windows and the like. Thus, the focused energy may be produced in a number of different ways, including other ways not mentioned here.

In another aspect, ablation device 14 is operated during two different time periods while varying at least one characteristic of ablation device 14, such as the frequency of the ablating energy, the power of the ablating energy, the position of the focus relative to the tissue, and/or the ablating time. For example, ablation device 14 may be operated at varying frequencies over time to ablate tissue in a controlled manner. Specifically, ablation device 14 is preferably operated to create a transmural lesion by controlling the delivery of energy to the tissue. Although it is preferred to vary the frequency when ablating the tissue, ablation device 14 may, of course, be operated at a single frequency.

In a first treatment method, the transducer is activated at a frequency of about 2 MHz to about 7 MHz, and preferably of about 3.5 MHz, and a power of about 80 watts to about 150 watts, and preferably of about 130 watts, in short bursts. For example, the transducer may be activated for about 0.01 second to about 2.0 seconds, and preferably for about 1.2 seconds. The transducer is inactive for about 2 seconds to about 90 seconds, more preferably about 5 seconds to about 80 seconds, and most preferably about 45 seconds between activations. In this manner, a controlled amount of accumulated energy can be delivered to the tissue in short bursts to heat tissue at and near the focus while minimizing the impact of blood cooling at the FS. Ablation at this frequency may continue until a controlled amount of energy is delivered, such as about 0.5 kilojoule to about 3 kilojoules. Treatment at this frequency in relatively short bursts produces localized heating at the focus. At the first frequency, energy is not absorbed as quickly in the tissue as it is at higher frequencies, so that heating at the focus is not significantly affected by absorption of ultrasound energy in tissue before reaching the focus.

Following treatment at the first frequency, the transducer is operated for longer periods of time, preferably about 1 second to about 4 seconds, and more preferably about 2 seconds, to ablate tissue between the focus and the transducer. The frequency during this treatment is also preferably about 2 MHz to about 14 MHz, more preferably about 3 MHz to about 7 MHz, and most preferably about 6 MHz. The transducer is operated for about 0.7 second to about 4 seconds at a power of about 20 watts to about 80 watts, and preferably about 60 watts. The transducer is inactive for between about 3 seconds and about 60 seconds, and preferably for about 40 seconds, between each activation. In this manner, a controlled amount of energy can be delivered to heat tissue between the focus and the transducer. The treatment at this frequency may continue until a controlled amount of total energy is delivered, such as about 750 joules.

As a final treatment, the ultrasonic transducer is activated at a higher frequency to heat and ablate the NS. The transducer is preferably operated at a frequency of between about 3 MHz and about 16 MHz, and preferably at about 6 MHz. The transducer is operated at lower power than the treatment methods above since the ultrasonic energy is rapidly absorbed by the tissue at these frequencies, so that the NS is heated quickly. In a preferred method, the transducer is operated at about 2 watts to about 20 watts, and more preferably about 15 watts. The transducer is preferably operated for a sufficient duration to ablate tissue, such as about 20 seconds to about 80 seconds, and preferably about 40 seconds. Often, the NS temperature will reach about 70 degrees C to about 85 degrees C.

Each of the treatments described above may be used by itself or in combination with other treatments. Furthermore, the combination of transducer size, power, frequency, activation time, and focal length may all be varied to produce the desired delivery of ultrasound energy to the tissue. As such, it is understood that the preferred embodiment may be adjusted by adjusting one or more of the characteristics. The treatment sequence described above generally delivers energy closer to the NS during the second treatment and even closer to the NS for the third treatment (that is, it ablates tissue from the FS towards the NS in successive treatments).

The focus of the ultrasound energy may also be moved relative to the tissue to deliver energy to different depths in the tissue. Ablation device 14 can be moved closer to and farther away from the target tissue, with the membrane conforming to the required shape to fill the gap between the transducer and the tissue. The membrane is preferably inflated, for example utilizing a fluid such as saline, and deflated to move the focus. However, ablation device 14 may also be moved with any other suitable mechanism, such as a threaded foot.

The focus may be moved while ablation elements 26 are activated or may be moved between activations of ablation elements 26. Moving the focus of the ultrasound energy may be sufficient to create a transmural lesion without changing frequencies, or may be used in conjunction with a change in frequencies as described above. The focus may also be moved in any other manner such as with a phased array or variable acoustic lensing.

After ablation elements 26 have been activated to ablate tissue, it may be necessary to ablate tissue in gaps between ablations from each ablation element 26. In one method of ablating these gaps, the entire ablation device 14 is shifted so that at least some ablation elements 26 are positioned to ablate tissue within one or more gaps. Thus, after first ablating tissue with all of the ablation elements 26, ablation device 14 is shifted and at least some, and preferably all, ablation elements 26 are activated again to create a substantially continuous lesion.

Another method to ablate tissue within gaps is to tilt ablation elements 26 to ablate tissue within gaps. In this method, ablation device 14 does not need to be moved. Rather, the membrane may be inflated to tilt the transducer, which directs the ultrasound energy toward tissue within gaps between transducers.

In another embodiment, ablation elements 26 may be located along a track such that one or more ablation elements 26 may be adjusted or moved (for example, by sliding) along the track so that any gaps in the ablation may be filled in by an activation of ablation elements 26 after they have been resituated over any such gaps. The use of sliding elements 26 may also be used to reduce the number of overall ablation elements 26 that are needed for an ablation procedure. For example, if sizing measurements (e.g., with introducer 20) reveal that an appropriately sized ablation device 14 would require 20 ablation elements 26, an ablation device 14 having 10 or fewer ablation elements 26 could be used, provided the 10 ablation elements 26 are adjustable along the track in order to complete the ablation annulus. Preferably, the track could be made using a superelastic material, including for example, a memory metal such as Nitinol. For example, all of the ablation elements 26 may be interconnected using one or more tracks of Nitinol or another superelastic material, such that ablation device 14 may be straightened for insertion into a patient and thereafter manipulated into a predetermined curvature to facilitate manipulations around the heart.

When the track is formed of superelastic material, the track not only permits ablation elements 26 to move therealong, it also permits ablation device 14 to achieve two different configurations. As described above, the superelastic properties allow ablation device 14 to be deformed such that ablation elements 26 are substantially coplanar, thereby allowing ablation device 14 to be straightened for insertion and guiding through a small incision, and then returning to the predetermined curvature when manipulated about the heart.

The track itself, or an isolated channel in the track, may also permit transmission of control signals from controller 12 that are used to control the operation of ablation elements 26 positioned along the track. These control signals may be used to reposition ablation elements 26 along the track or otherwise alter the ablating energy being delivered to the tissue.

Controller 12 may be designed to automatically ablate in any manner described herein. For example, controller 12 can change the frequency, power, focal length, and/or operating time to provide the desired ablating technique. The change in frequency and power may be completely automatic or may require some user input such as visual indications of fat and/or tissue thickness. For example, controller 12 may be designed to automatically sequence through two or more different ablating techniques such as those described above. Other techniques, of course, may be used depending on the tissue characteristics and the type and characteristics of the one or more ultrasound transducers. Controller 12 may also utilize feedback, such as temperature feedback or electrical impedance, to actively control the ablations.

Although multiple embodiments have been described above with a certain degree of particularity, those skilled in the art could make numerous alterations to the disclosed embodiments. For example, although the ablation device has been described in connection with creating a substantially continuous lesion around all pulmonary veins, it should be understood that the methods disclosed herein are equally applicable to ablating only partially around the pulmonary veins. Furthermore, other lesions may be beneficial in treating electrophysiological conditions, and the devices and methods described herein may be useful in creating such lesions on other parts of the heart and in other areas of the body. It should also be understood that a wand-type device may be used in conjunction with the invention disclosed herein during an ablation procedure, for example to create a mitral isthmus ablation lesion contiguous with the PV isolation lesion or to fill in any gaps in the PV isolation lesion created by ablation device 14.

It is intended that all matter contained in the above description or shown in the accompanying drawings shall be interpreted as illustrative only and not limiting. Changes in detail or structure may be made without departing from the scope the invention as defined in the appended claims.

## Claims

1. An introducer adapted to introduce an ablation device in the area to be treated an to size an area or at least a portion of a circumference around one or more pulmonary veins along an epicardial surface, the introducer comprising:
an elongated body having a distal end (70), a proximal end (68), and an intermediate portion (72) therebetween, the distal end (70) being configured to form at least a partial loop when in a resting, unbiased shape(68) and said elongated body having an insert located at one of the distal end and the intermediate portion (72);
a handle (64) located at the proximal end of the elongated body; and
a shaft (62) rigidly coupled to the handle (64) and extending to the insert of the elongated body to assist in transferring torque from the handle to at least one of the distal end and the intermediate portion (72) or the elongated body.

2. The introducer of claim 1, wherein
said elongated body including a lumen (61) extending from the proximal end (68) to a point within the intermediate portion (72);
said insert is at least one receptacle (72) accessible via the lumen (61), elongated body; and
the shaft (62) is fixedly coupled to the handle (64) and extending from the handle (64) to the at least one receptacle (72) to assist in transferring torque from the handle (64) to the intermediate portion (72).

3. The introducer of claim 2, wherein the elongated flexible body of the introducer (20) comprises at least two receptacles (73) and said shaft (62) includes at least two ends that are configured to fit within the at least two receptacles (72).

4. The introducer of any preceding claim, further comprising a plurality of sizing indicators (24) along a length of the elongated body, wherein the plurality of sizing indicators (24) are indicative of a distance that is being measured by the at least a partial loop formed near the distal end (70) of the introducer.

5. THe introducer of any preceding claim wherein the proximal end (68) of the introducer (20) further comprises a connector that is configured to receive the handle (64).

6. The introducer of claim 5; wherein the proximal end (68) of the introducer (20) further comprises a snap-fit (46) connector that is configured to receive the handle (64).

7. The introducer of any preceding claim, wherein a cross section of the elongated flexible body at the proximal end (68) is larger than a cross section of the elongated flexible body at the distal end (70).

8. The introducer of any preceding claim, wherein the elongated body of the introducer (20) further comprises a braided wire assembly (71) that extends from the proximal end (68) toward the distal end (70).

9. The introducer of any preceding claim, wherein the shaft (62) is made of a material that resists twisting forces.

10. The introducer of any according claim, wherein the shaft (62) comprises steel.

11. The introducer of any preceding claim, wherein the insert comprises a groove and the shaft (62) terminates in a flattened head that mates with the groove.

12. A kit for use in a lateral thoracotomy procedure comprising:
an introducer (20), according to any of preceding claims comprising:
an elongated flexible body having a proximal end (68), a distal end (70), and an intermediate portion (72) therebetween,
wherein the distal end (70) forms at least a partial loop in a relaxed state,
said elongated flexible body also including a lumen (61) extending therethrough from the proximal (68) and to to a point between the proximal end (68) and a point at which the distal end (70) begins to curve,
said elongated flexible body also including at least one torque imparting receptacle (73) accessible via the lumen, said receptacle (73) being configured to impart torque onto the intermediate portion (72) of the elongated body: and
a torque transfer device (60) comprising a handle (67) and a shaft (62) fixedly coupled to the handle (64), said shaft (62) being configured to be inserted through the lumen (61) and into the at least one receptacle (73), whereby torque imparted to the handle (64) is transferred to the intermediate portion (72) of the introducer (20) via the shaft (62).

## Patentansprüche

1. Inserter, der dazu angepasst ist, eine Abtragungsvorrichtung in den zu behandelnden Bereich einzuführen und einen Bereich oder wenigstens einen Umfangsabschnitt um eine oder mehrere Lungenvenen entlang einer epikardialen Fläche abzumessen, wobei der Inserter aufweist:
einen länglichen Körper mit einem distalen Ende (70), einem proximalen Ende (68) und einem dazwischen angeordneten mittleren Abschnitt (72), wobei das distale Ende (70) dazu ausgebildet ist, wenigstens eine Teilschlaufe zu bilden, wenn diese in einer ruhenden, nicht vorgespannten Form ist, und der längliche Körper einen Einsatz aufweist, der an dem distalen Ende (70) oder dem mittleren Abschnitt (72) angeordnet ist;
einen am proximalen Ende des länglichen Körpers angeordneten Handgriff (64); und
einen Schaft (62), der fest mit dem Handgriff (64) verbunden ist und sich zum Unterstützen beim Übertragen von Drehmoment vom Handgriff (64) zum distalen Ende (70) und/oder zum mittleren Abschnitt (72) des länglichen Körpers zu dem Einsatz des länglichen Körpers erstreckt.

2. Inserter nach Anspruch 1, bei dem
der längliche Körper ein Lumen (61) aufweist, das sich von dem proximalen Ende (68) zu einem Punkt innerhalb des mittleren Abschnitts (72) erstreckt;
der Einsatz wenigstens ein über das Lumen (61) zugänglicher Stecker (73) ist, wobei der Stecker (73) dazu ausgebildet ist, ein Drehmoment in den mittleren Abschnitt (72) des länglichen Körpers einzubringen; und
der Schaft (62) fest mit dem Handgriff (64) verbunden ist und sich zum Unterstützen beim Übertragen von Drehmoment vom Handgriff (64) zum mittleren Abschnitt (72) vom Handgriff (64) zu dem wenigstens einen Stecker (73) erstreckt.

3. Inserter nach Anspruch 2, bei dem der längliche, flexible Körper des Inserters (20) wenigstens zwei Stecker (73) umfasst und der Schaft (62) wenigstens zwei Enden aufweist, die dazu ausgebildet sind, in die wenigstens zwei Stecker (73) zu passen.

4. Inserter nach einem der vorhergehenden Ansprüche, ferner mit einer Mehrzahl von Messindikatoren (24) entlang einer Länge des länglichen Körpers, wobei die Mehrzahl von Messindikatoren (24) eine Distanz anzeigen, die von der wenigstens einen Teilschlaufe, die in der Nähe des distalen Endes (70) des Inserters gebildet ist, gemessen wird.

5. Inserter nach einem der vorhergehenden Ansprüche, bei dem das proximale Ende (68) des Inserters (20) ferner einen Verbinder aufweist, der dazu ausgebildet ist, den Handgriff (64) zu empfangen.

6. Inserter nach Anspruch 5, bei dem das proximale Ende (68) des Inserters ferner einen Schnappverbinder (46) aufweist, der dazu ausgebildet ist, den Handgriff (64) zu empfangen.

7. Inserter nach einem der vorhergehenden Ansprüche, bei dem ein Querschnitt des länglichen, flexiblen Körpers am proximalen Ende (68) größer als ein Querschnitt des länglichen, flexiblen Körpers am distalen Ende (70) ist.

8. Inserter nach einem der vorhergehenden Ansprüche, bei dem der längliche Körper des Inserters (20) ferner eine geflochtene Drahtanordnung (71) aufweist, die sich vom proximalen Ende (68) in Richtung des distalen Endes (70) erstreckt.

9. Inserter nach einem der vorhergehenden Ansprüche, bei dem der Schaft (62) aus einem Material besteht, das Verwindungskräften standhält.

10. Inserter nach einem der vorhergehenden Ansprüche, bei dem der Schaft (62) aus Stahl besteht.

11. Inserter nach einem der vorhergehenden Ansprüche, bei dem der Einsatz eine Kerbe aufweist und der Schaft (62) in einem flachen Kopf endet, der mit der Kerbe zusammenpasst.

12. Bausatz zum Verwenden in einer lateralen Thorakotomieprozedur, mit:
einem Inserter (20) nach einem der vorhergehenden Ansprüche, aufweisend:
einen länglichen, flexiblen Körper, der ein proximales Ende (68), ein distales Ende (70) und einen dazwischen angeordneten mittleren Abschnitt (72) aufweist,
bei dem das distale Ende (20) wenigstens eine Teilschlaufe in einem Ruhezustand bildet, wobei der längliche, flexible Körper ferner ein Lumen (61) aufweist, das sich dorthindurch vom proximalen Ende (68) zu einem Punkt zwischen dem proximalen Ende (68) und einem Punkt erstreckt, an dem das distale Ende (70) beginnt, sich zu krümmen,
wobei der längliche, flexible Körper ferner wenigstens einen über das Lumen (61) zugänglichen Drehmomenteinbringstecker (73) umfasst, wobei der Stecker (73) dazu ausgebildet ist, ein Drehmoment in den mittleren Abschnitt (72) des länglichen Körpers einzubringen; und
eine Drehmomentübertragungsvorrichtung (60) mit einem Handgriff (64) und einem Schaft (62), der fest an dem Handgriff (64) befestigt ist, wobei der Schaft (62) dazu ausgebildet ist, durch das Lumen (61) und in den wenigstens einen Stecker (73) eingerührt zu werden, wobei ein in den Handgriff (64) eingebrachtes Drehmoment über den Schaft (62) zum mittleren Abschnitt (72) des Inserters (20) übertragen wird.

## Revendications

1. Introducteur adapté pour l'introduction d'un dispositif d'ablation dans la zone à traiter et pour le calibrage d'une zone et/ou au moins d'une portion d'une circonférence autour d'une ou de plusieurs veines pulmonaires le long d'une surface épicardique, l'introducteur comprenant :
un corps allongé ayant une extrémité distale (70), une extrémité proximale (68) et une portion intermédiaire (72) entre celles-ci, l'extrémité distale (70) étant configurée pour former au moins une boucle partielle lorsqu'elle est dans une configuration assignée non biaisée, ledit corps allongé ayant un insert situé à l'une de l'extrémité distale (70) et de la portion intermédiaire (72) ;
une poignée (64) située à l'extrémité proximale du corps allongé ; et
une tige (62) couplée rigidement à la poignée (64) et s'étendant jusqu'à l'insert du corps allongé pour faciliter le transfert du couple de la poignée (64) vers au moins l'une de l'extrémité distale (70) et de la portion intermédiaire (72) du corps allongé.

2. Introducteur selon la revendication 1, dans lequel :
ledit corps allongé incluant une lumière (61) s'étendant de l'extrémité proximale (68) vers un point situé dans la portion intermédiaire (72) ;
ledit insert est au moins un réceptacle (73) accessible par la lumière (61), ledit réceptacle (73) étant configuré pour transmettre le couple à la portion intermédiaire (72) du corps allongé ; et
la tige (62) est couplée fixement à la poignée (64) et s'étend de la poignée (64) vers au moins un réceptacle (73) pour faciliter le transfert du couple de la poignée (64) à la portion intermédiaire (72).

3. Introducteur selon la revendication 2 dans lequel le corps flexible allongé de l'introducteur (20) comprend au moins deux réceptacles (73), ladite tige (62) comprenant au moins deux extrémités qui sont configurées pour s'adapter dans les au moins deux réceptacles (73).

4. Introducteur selon l'une quelconque des revendications qui précèdent, comprenant en outre une pluralité d'indicateurs de calibrage (24) sur une longueur du corps allongé, dans lequel la pluralité d'indicateurs de calibrage (24) est indicatrice d'une distance qui est mesurée par au moins une boucle partielle formée à proximité de l'extrémité distale (70) de l'introducteur.

5. Introducteur selon l'une quelconque des revendications qui précèdent, dans lequel l'extrémité proximale (68) de l'introducteur comprend en outre un connecteur qui est configuré pour recevoir la poignée (64).

6. Introducteur selon la revendication 5, dans lequel l'extrémité proximale (68) de l'introducteur comprend en outre un connecteur à encliquetage (46) qui est configuré pour recevoir la poignée (64).

7. Introducteur selon l'une quelconque des revendications qui précèdent, dans lequel une coupe transversale du corps flexible allongé à l'extrémité proximale (68) est plus grande qu'une coupe transversale du corps flexible allongé à l'extrémité distale (70).

8. Introducteur selon l'une quelconque des revendications qui précèdent, dans lequel le corps allongé de l'introducteur comprend en outre un ensemble tressé (71) qui s'étend de l'extrémité proximale (68) vers l'extrémité distale (70).

9. Introducteur selon l'une quelconque des revendications qui précèdent, dans lequel la tige (62) est fabriquée dans un matériau qui résiste aux forces de torsion.

10. Introducteur selon l'une quelconque des revendications qui précèdent, dans lequel la tige (62) comprend de l'acier.

11. Introducteur selon l'une quelconque des revendications qui précèdent, dans lequel l'insert comprend une rainure et la tige (62) se termine par une tête aplatie qui s'accouple avec la rainure.

12. Kit pour utilisation dans une procédure de thoracotomie latérale comprenant :
un introducteur (20), selon l'une quelconque des revendications qui précèdent, comprenant :
un corps flexible allongé ayant une extrémité proximale (68), une extrémité distale (70) et une portion intermédiaire (72) entre celles-ci,
dans lequel l'extrémité distale (70) forme au moins une boucle partielle dans un état détendu,
ledit corps flexible allongé incluant également une lumière (61) s'étendant à travers celui-ci de l'extrémité proximale (68) au point situé entre l'extrémité proximale (68) et un point où l'extrémité distale (70) commence à se courber,
ledit corps flexible allongé incluant également au moins un réceptacle (73) transmettant le couple, accessible par la lumière, ledit réceptacle (73) étant configuré pour transmettre le couple à la portion intermédiaire (72) du corps allongé ; et
un dispositif de transfert du couple (60) comprenant une poignée (67) et une tige (62) couplée fixement à la poignée (64), ladite tige (62) étant configurée pour être insérée à travers la lumière (61) et dans le au moins un réceptacle (73), grâce à quoi le couple transmis à la poignée (64) est transféré à la portion intermédiaire (72) de l'introducteur (20) par la tige (62).
